# EUROPEAN PATENT APPLICATION

(11) **EP 3 685 776 A1**
(43) Date of publication of application: **29.07.2020**
(21) Application number: 19305103.4
(22) Date of filing: 25.01.2019
(51) Int. Cl.: A61B 17/34

(54) **HOLDING SYSTEMS FOR MEDICAL INSTRUMENTS**

(71) Applicant: CHU de NICE, 06200 Nice (FR)
(72) Inventor: SEJOR, Eric, 06000 Nice (FR)
(74) Representative: Osha Liang

(57) **Abstract**

The present disclosure concerns a system (4) for holding a medical instrument (21; 22) comprising a male member (41) comprising at least one male thread (416) and at least one first axial lumen (411) and a female member (42) comprising at least one female thread (426) and at least one second axial lumen (421) When the at least one male thread (416) and the at least one female thread (426) are engaged to each other, the at least one first axial lumen (411) and the at least one second axial lumen (421) form a common axial lumen (411, 421) for at least partially receiving a medical instrument (21; 22). The system (4) further comprises a locking means (412, 427) for locking the medical instrument (21; 22) in the common axial lumen (411, 421) at a predetermined position.

## Description

### Field of the disclosure

The present disclosure concerns, but is not limited to, systems for holding medical instruments and, more particularly, to systems and methods for holding a medical instrument in a trocar that may be percutaneously inserted into a patient's body.

### Technical background

Surgery requires increased access to internal tissues through minimally invasive surgical procedures. The term "minimally invasive" refers to a wide range of surgical procedures including laparoscopic, thoracoscopic, arthroscopic, endoscopic, and natural orifice transluminal procedures.

During some minimally invasive procedures, trocars percutaneously inserted into a patient's body may be used to provide a port for medical instruments.

Development in minimally invasive surgery has resulted in increasingly complex procedures requiring the use of numerous medical instruments, as well as precise manipulations within the body. The weight of medical instruments and even trocars often requires additional surgeons in order to uphold the position of instruments.

Accordingly, there is a need for systems and methods that allow to maintain a medical instrument at a desired position, thereby freeing users for other tasks or even giving the opportunity for one to operate alone.

### Summary of the disclosure

In what follows, the term "comprise" is synonym of (means the same as) "include" and "contains", is inclusive and open and does not exclude other non-recited elements.

According to a first aspect, the present disclosure concerns a system for holding a medical instrument, comprising: a male member comprising at least one male thread and at least one first axial lumen; a female member comprising at least one female thread and at least one second axial lumen; wherein, when the at least one male thread and the at least one female thread are engaged to each other, the at least one first axial lumen and the at least one second axial lumen form a common axial lumen suitable for at least partially receiving a medical instrument; and wherein said system further comprises: a locking means for locking the medical instrument in the common axial lumen at a predetermined position.

The system according to the disclosure allows, in a simple way, to hold a medical instrument in a trocar which may be percutaneously inserted into a patient's body. By holding, it is meant that the medical instrument is at least partially impeded in its movement. Said movement may comprise an axial and/or radial and/or tangential movement with respect to the common axial lumen edge, and the degree of impediment may vary. By locking, it is meant that any movement of the medical instrument is totally impeded within the system. Said system may be used, for example, during a surgical intervention. The system according to the disclosure may then afford, among others, the following advantages:
- the liberation of hands: the medical instrument, once correctly positioned within the body cavity of a patient by a surgeon, can be locked at a predetermined position in the common axial lumen of the system according to the disclosure, and therefore be stabilized at a predetermined position. If the medical instrument is meant to be at least partially inserted in a trocar, for example, the system according to the disclosure allows to stabilize and secure the medical instrument within the trocar while the instrument is not manipulated by the surgeon. Hence, the medical instrument does not have to be physically held by a member of a surgical team throughout the whole duration of a surgical intervention. Less human assistance is then needed, which facilitates and lowers the costs of the intervention.
- the surgeon's access to the patient's body may be less hindered. As less operators may be needed to hold the medical instrument in place, more available space may then be cleared.

According to one or more embodiments, in order to form a common axial lumen for at least partially receiving a medical instrument, the at least one first axial lumen and the at least one second axial lumen may be axially aligned. According to one or more embodiments, both the at least one first axial lumen and the at least one second axial lumen are axially aligned, cylindrical, and have the same diameter. Hence, in these embodiments, when the male member and the female member are connected to each other, both lumens may form a unique same lumen as the common axial lumen.

The medical instruments that are susceptible to being held by the system according to the disclosure may be any medical instrument which needs to be held at a predetermined position. According to one or more embodiments, the medical instrument is selected from the group comprising surgical instruments, diagnostic instruments. By surgical instrument, it is meant any type of instrument used for surgery. According to one or more embodiments, the medical instrument is selected from the group comprising aspiration instruments, cannulas, carrier bags, cautery hooks, clip appliers, graspers, endoloops, staplers, needle drivers, retractors, scissors, vessel sealers. According to one or more embodiments, the medical instrument comprises a grasper, that may for example be fenestrated, that may be short or long, that may be flat or curved. For example the grasper may be a long curved grasper. According to one or more embodiments, the medical instrument comprises a pair of scissors, for example a pair of ultrasonic scissors. According to one or more embodiments, the medical instrument is a retractor, such as for example a five retractor. According to one or more embodiments, the medical instrument comprises a stapler, for example a linear stapler. The above-mentioned medical instruments are cited in a non-limiting, illustrative manner.

According to one or more embodiments, the medical instrument may comprise a shaft. According to one or more embodiments, said shaft might be a tube, such as for example the tube of an endoscopic camera.

According to one or more embodiments, the at least one male thread and the at least one female thread may be engaged to each other by screwing the female member onto the male member.

According to one or more embodiments, the locking means is configured to set the system according to the disclosure in any of a plurality of intermediate positions between an open position, at which the medical instrument freely moves in the common axial lumen, and a closed position, at which the medical instrument is locked at the predetermined position. According to one or more embodiments, the plurality of intermediate positions can be a continuum, i.e. a homogeneous continuity of intermediate positions, as opposed to a discontinuity of intermediate punctual positions.

According to one or more embodiments, the locking means and/or the female thread and male thread are configured such that the medical instrument at least partially received in the common axial lumen is locked at a predetermined position at a predetermined degree of engagement of the at least one male thread and the at least one female thread to each other. According to one or more embodiments, the degree of thread engagement is correlated to the positions at which the system can be set, be they open, intermediate, or closed. The resulting simplicity of actuation of the instrument's holding allows the surgeon to ergonomically open or close the system, for example by using his fingers and without rotating the wrist.

According to one or more embodiments, predetermined degrees of thread engagement may be selected in correlation with the plurality of positions at which the system may be set, the latter being provided by the configuration of the locking means. According to one or more embodiments, the correlation between predetermined degrees of thread engagement and the plurality of positions may be continuous. This means that the continuity provided by engaging the at least one male thread to the at least one female thread, which is inherent to the threads, may be arranged to correspond to a continuity on the locking action of the locking means, thereby affording the possibility to set the system in a continuity of intermediate positions between the open and closed positions, simply by screwing. The resulting cooperative continuity between the threads and the locking means offers, among other advantages, the possibility for a surgeon to easily replace a medical instrument of one type, initially held in the system according to the disclosure, by another instrument of another type. Indeed, a plurality of medical instruments may be used during a surgical intervention and it might be needed to insert more than one medical instrument and/or more than one type of medical instrument within the same trocar at different stages of a surgical procedure. Hence, according to one or more embodiments, the cooperative continuity between the threads and the locking means according to one or more embodiments can afford, besides the liberation of the surgeon's or other operators' hands during a surgical procedure, a flexibility of the system, which can hold medical instruments with different sizes, without necessitating any preadjustment or preselection of the intermediate position that allows insertion and holding and/or locking of an instrument with a given standard size. Accordingly, the holding system according to one or more of these embodiments can be seen as an auto-adjusting system, compared to other systems for which the positions of the locking means are pre-adjusted to the specific sizes of standard instruments.

According to one or more embodiments, the locking means is integral with the male member and/or the female member. For example, at least one part of the locking means may be integral with the male member and at least another part of the locking means may be integral with the female member. According to one or more embodiments, the locking means is integral with the male member and/or the female member and the system may consist only of said male and female members.

Such a system has a small number of pieces, which offers simplicity of manufacturing and/or a low price of manufacture. Likewise, such a system can be easily assembled, easily disposable, easily sterilized. Such systems which may be reusable but also disposed after each use are attractive to surgeons.

According to one or more embodiments, the locking means comprises at least one of a blocking wing, a beveled cylindrical wall, a jaw, a wedge, a spring, and combinations thereof.

The male member, the female member and the locking means may be made from any number of materials. The selection of the nature of materials used to manufacture the system may result from a compromise between robustness and flexibility. For example, certain parts of the locking means might be entrained to bend upon engaging the at least one male thread to the at least one female thread. Flexibility and/or rigidity of the materials may be desirable and their choice may depend on certain requirements of the surgeon, for example in order to get a system able to repeatedly positioning a medical instrument and capable of stabilizing it once positioned at one of a number of predetermined positions, and/or a system privileging the stability of the locking. The robustness of the system as a whole may for example depend on the physical constraints applied on the different parts of the system by the medical instrument.

Moreover, according to one or more embodiments, the locking means may comprise a flexible material. The presence of a flexible material within the locking means may offer a further advantage to the system. For example, if the locking means sets the system in an intermediate position which is close to the closed position within the above-mentioned plurality of intermediate positions, while the medical instrument is held in the system, if needed, the surgeon can axially slide the medical instrument along the common axial lumen without having to simultaneously engage the at least one male thread to the at least one female thread and hold the medical instrument.

Merely as examples and not as an exhaustive list, materials used to manufacture the system according to one or more embodiments may include metals such as stainless steel or Nitinol or may include plastics like nylon, polyvinylchloride or polypropylene. Such materials may be formed by laser cutting, molding, welding, brazing or other processes known in the art.

According to one or more embodiments, the locking means is configured to apply a radial and/or an axial constraint on the medical instrument. According to one or more embodiments, such radial and/or axial constraint may be applied by engaging the at least one male thread and the at least one female thread to each other. According to one or more embodiments, such radial and/or axial constraint may be induced by friction between the locking means and the part of the medical instrument in contact with the locking means. For example, the radial forces at stake in a radial constraint may allow to avoid high frictional forces, which might damage the locking means, thereby rendering the system little or non-reusable.

According to one or more embodiments, the male member is either reversibly attachable to a trocar or integrally forms part of a trocar. According to one or more embodiments, the female member is either reversibly attachable to a trocar or integrally forms part of a trocar. According to one or more embodiments, the male member and the female member integrally form part of a trocar. For example, the male member may be integral with a trocar, with the female member coming as a detachable piece. In alternative embodiments, the male member or the female member may comprise a part adapted to be attached to a trocar, thereby rendering the system completely detachable from the trocar. For example, the male member or the female member may comprise a plurality of fixation hooks for attaching the system to a trocar. In some embodiments, such part adapted to be attached to a trocar may be adapted to be attached to a large number of different trocars, thereby conferring a universal character to the system.

According to one or more embodiments, the system comprises a gas tight diaphragm or gasket between the male member and the female member. Said diaphragm or gasket may for example be composed of elastomeric material. Said diaphragm or gasket may include an annular lip, thanks to which an even frictional force is encountered, irrespective of the size of the medical instrument's part intended to be received in the common axial lumen. Such an arrangement provides a gas tight seal through which the medical instrument may pass.

Other advantages might be attained by the system according to one or more embodiments. For example, different colors may be used according to the thread type, the size of the common axial lumen and/or the size of the medical instrument(s). For example, a color code might be used so that the surgeon can easily discriminate between different sizes of threads, which he/she knows are directly correlated to predetermined ranges of medical instruments' sizes. At least a part of the external surface of the system according to one or more embodiments may be cylindrical, which renders said holding system easy to manually actuate by a surgeon during use. The holding system according to the disclosure may also comprise a top cover, for example formed from transparent waterproof material, arranged to prevent occlusion of the moving parts of the system and/or ingress of dirt during use.

According to a second aspect, the present disclosure concerns a trocar attachable to or integral with the male member or the female member of the system according to the first aspect. According to one or more embodiments, the trocar may comprise the male member or the female member as an integral part of itself. According to other embodiments, the trocar may be configured to be attachable to the male member or the female member. In embodiments wherein the member, be it male or female, comprises a plurality of fixation hooks, said trocar may for example comprise a plurality of notches receiving said fixation hooks.

According to a third aspect, the present disclosure concerns a method for locking a medical instrument in a trocar, comprising:
- providing a system according to the first aspect or any of the embodiments thereof;
- at least partially inserting a medical instrument in the common axial lumen of the system;
- engaging the at least one male thread with the at least one female thread to each other to hold the medical instrument in a trocar; and
- locking the at least partially received medical instrument in the common axial lumen at a predetermined position.

The embodiments described above are not exhaustive. In particular, it is understood that additional embodiments can be considered on the basis of different combinations of the explicitly described embodiments. Unless otherwise specified in the present disclosure, it will be apparent to the skilled person that all the embodiments described above can be combined together. For example, unless otherwise specified, all features of the embodiments described above, whether they refer to the locking means, the male member, or the female member, can be combined with or replaced by other features from other embodiments.

### Brief description of the drawings

The present disclosure will be better understood and other advantages and embodiments will become clear on reading the description that follows, given purely by way of indication and in no way limiting, and by referring to the appended figures
in which:
- FIG. 1 is a perspective view of different medical instruments held by systems according to one or more embodiments of the present disclosure, said systems being attached to trocars inserted percutaneously into a patient's body.
- FIG. 2A is a perspective view of a system according to one or more embodiments of the present disclosure.
- FIG. 2B is a perspective exploded view of the system of FIG. 2A.
- FIG. 3A-3C are a top, bottom and perspective views, respectively, of the female member of the system of FIG. 2A.
- FIG. 4A-4D are a top, bottom, side and perspective views, respectively, of the male member of the system of FIG. 2A.

### Detailed description of embodiments

Embodiments of the present disclosure will now be described in detail with reference to the accompanying figures. In the following detailed description of embodiments of the present disclosure, numerous specific details are set forth in order to provide a more thorough understanding of the present disclosure. However, it will be apparent to one of ordinary skill in the art that the present disclosure may be practiced without these specific details. In other instances, well-known features have not been described in detail to avoid unnecessarily complicating the description.

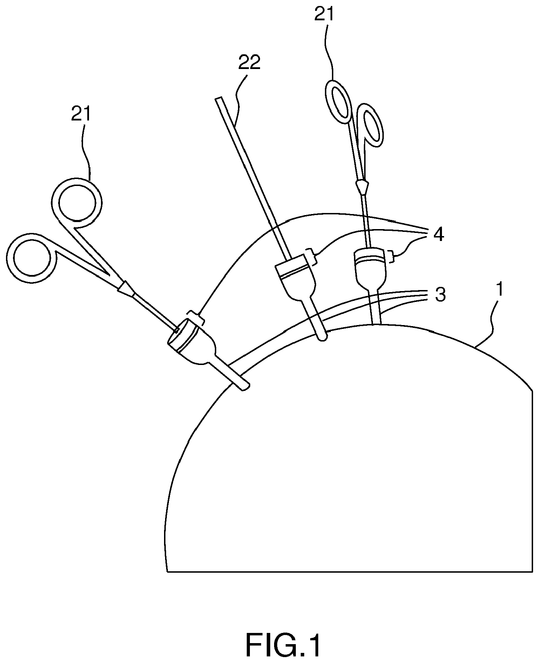

The following description provides non-limiting examples of a system according to one or more embodiments of the present disclosure, which in FIG. 1 is designated by reference number 4.

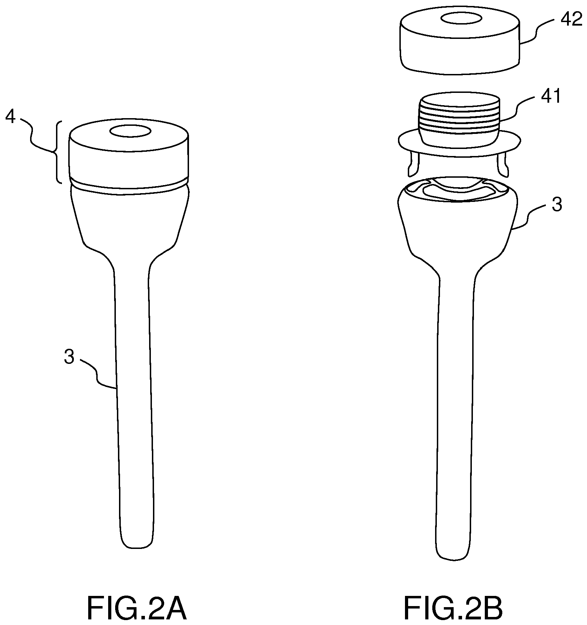

The system 4, in the same manner as other examples of systems according to any of the embodiments of the present disclosure, may serve as an efficient medical instruments' holder. FIG. 1 depicts a use in surgery of said system 4, and FIGs. 2-4 show said system 4 with a further level of detail.

Three systems 4 are shown in FIG. 1, attached to their respective trocars 3, said trocars 3 being percutaneously inserted into a patient's body 1. Two types of medical instruments are depicted in FIG. 1, i.e., graspers 21 and an endoscopic camera 22. Both types of medical instruments, as well as any other medical instruments, may be held by the systems 4, in a relatively narrow space on the patient's body surface 1. This demonstrates the gain in precious available space around the medical instruments that is offered by systems according to the disclosure.

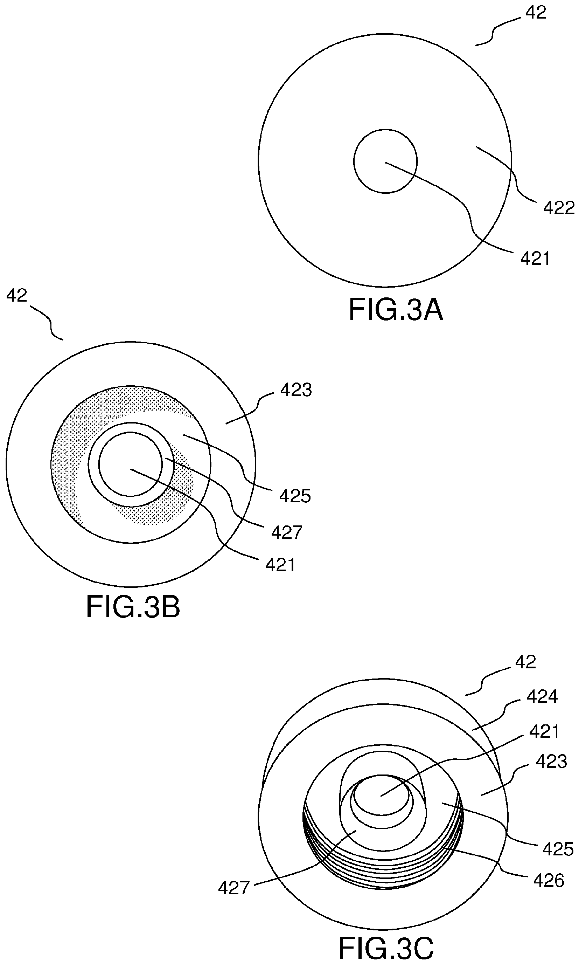

As shown in FIGs. 2A and 2B, and in line with the present disclosure, the system 4 comprises a male member 41 and a female member 42. As discussed in more detail with reference to FIGs. 3A-C, in accordance with the present disclosure, the male member 41 comprises one male thread and one first axial lumen. As discussed in more detail with reference to FIGs. A-D, the female member 42 comprises one female thread and one second axial lumen. When the male thread and the female thread are engaged to each other, the first axial lumen and the second axial lumen form a common axial lumen for at least partially receiving a medical instrument. The system 4 further comprises a locking means for locking the medical instrument in the common axial lumen at a predetermined position.

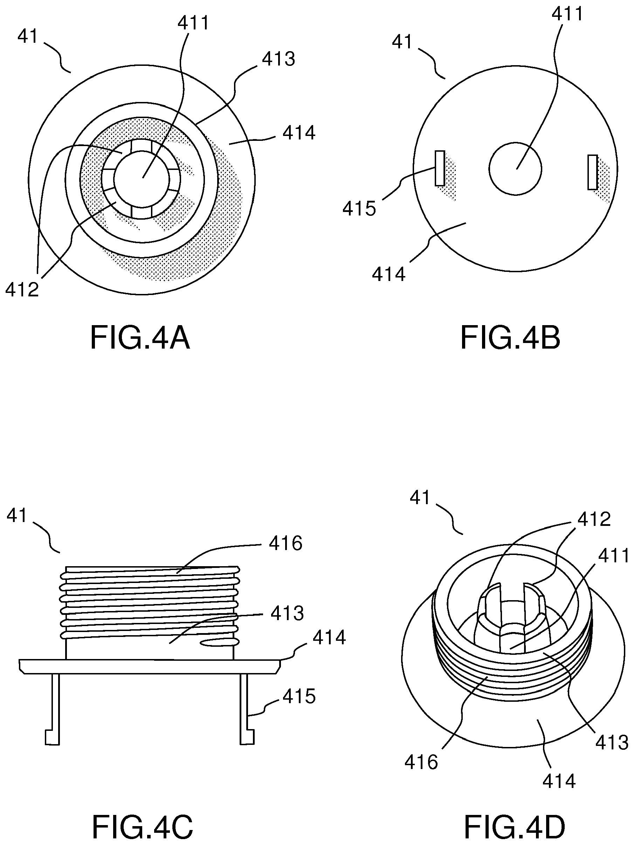

Certain parts of the system 4 are shown in more details in FIGs. 3A-C and FIGs. 4A-D, such as the male thread and the first axial lumen, designated by reference numbers 416 and 411, respectively, the female thread and the second axial lumen, designated by reference numbers 426 and 421, respectively, and the locking means, designated by reference numbers 412 and 427. In the following, the common axial lumen formed by the first axial lumen 411 and the second axial lumen 421 will be designated by the reference numbers 411, 421 together. The locking means 412, 427 of the system 4 is configured to set the system 4 in any of a plurality of intermediate positions between an open position, at which the medical instrument, such as 21 or 22, freely moves in the common axial lumen 411, 421, and a closed position, at which the medical instrument is locked at the predetermined position. In the example of system 4, the locking means 412, 427 is configured to apply a radial constraint on the medical instrument. Also, the locking means 412, 427 of system 4 is integrally formed with the male member 41 and the female member 42. In the embodiment shown in the figures, the system 4 consists only of only two components, i.e., the male member 41 and the female member 42. However, according to one or more embodiments, the system may also comprise further components.

FIGs. 2A and 2B further show that system 4 comprises a fixation elements, which in this embodiment are included in the male member, adapted to be attached to the trocar 3. Accordingly, the depicted trocar 3 is attachable to the system 4. However, in one or more embodiments, fixation elements adapted to attach the system to the trocar may be provided in the female member.

FIG. 3A-3C are different views of the female member 42 of the system 4 depicted in FIGs. 2A and 2B. Said female member 42 comprises
- a hollow cylinder 425 comprising a first annular wall 422 provided with an opening defining an axial lumen 421, a second annular wall 423, and an outer cylindrical wall 424;
- a female thread 426 internally provided on the outer cylindrical wall 424; and
- a beveled inner cylindrical wall 427 concentric with the outer cylindrical wall 424 and projecting from the first annular wall 422 at the opening thereof, the inner cylindrical wall 427 defining an axial lumen 421 suitable for receiving medical instruments and a concentric space 425 between the beveled inner cylindrical wall 427 and the outer cylindrical wall 424.

Since the outer wall 424 of the female member 42 is cylindrical, the holding system 4 is easy to be manipulated by a surgeon during use.

FIG. 4A-4D show different views of the male member 41 of the system 4. The male member 41 comprises:
- an annular wall 414 provided with an opening defining an axial lumen 411 suitable for receiving medical instruments, and an outer cylindrical wall 413 externally provided with a male thread 416;
- a plurality of blocking wings 412 circumferentially arranged around the opening; and
- a plurality of fixation hooks 415 disposed on the annular wall 414.

As discussed above, the locking means of the system 4 is configured to set the system 4 in any of a plurality of intermediate positions between an open position, at which the medical instrument 21, 22 freely moves in the common axial lumen 411, 421, and a closed position, at which the medical instrument 21, 22 is locked at the predetermined position. In the embodiment presented, the locking means of the system 4 comprises the beveled inner cylindrical wall 427 of the female member 42 and the plurality of blocking wings 412 of the male member 41. This locking means of this embodiment of system 4 is thus a combination of portions of both the male member and the female member. However, in on ore more embodiments, different configurations and arrangements of the locking means are possible, wherein the locking means is integrally formed with only one of the female member or the male member.

Each blocking wing 412 of the plurality of blocking wings serves to radially engage a part of a medical instrument (such as for example any of instruments 21 or 22 shown in FIG. 1 or any other medical instrument). For example, each blocking wing 412 may radially engage the shaft of a medical instrument inserted in the system 4 and passing through the common axial lumen 411, 421 formed by the axial lumen 421 and the axial lumen 411 when the male the male thread 416 and the female thread 426 are engaged to each other.

The blocking wings 412 are circumferentially arranged around the axial lumen 411, and their number equals four in system 4, as can be seen in FIGS. 4A and 4D. The number and configuration of blocking wings 412 is, of course, not limited to that depicted in FIG. 4. While four blocking wings are depicted, embodiments employing as few as one blocking wing have been contemplated. Likewise, embodiments employing more blocking wings 412 have been contemplated and the number of used blocking wings is only limited by the engineering considerations of the particular embodiment.

Other configurations of the locking means may be envisaged. For example, the blocking wings might be comprised in the female member and the beveled inner cylindrical wall in the male member. According to one or more embodiments, the locking means of the system may be of a different type and may for example comprise wedges attached to springs, said springs being linked to the male member and said wedges being forced to engage the medical instrument by the action of a beveled inner cylindrical wall comprised in the female member.

FIGS. 2-4 show a correspondence of the male member 41 and the female member 42, which are symmetrically opposed to each other and have pieces which can interlock, such as the hollow cylinder 425 of the female member 42 and the outer cylindrical wall 413 of the male member 41. Likewise, the first and second axial lumens 411 and 421 have the same diameter and are axially aligned to form the common axial lumen 411, 421 of system 4 when the at least one male thread 416 and the at least one female thread 426 are engaged to each other.

Moreover, the system 4 shows a cooperative continuity between the threads and the locking means 412, 427 comprising the blocking wings 412 and the beveled ring 427. Indeed, as the female member 42 gets progressively connected to the male member 41 by screwing, the beveled ring 427 comes to bend the blocking wings 412 towards the center of the common axial lumen 411, 421. Thereby, the extremities of the blocking wings 412 come to engage a part of a previously inserted medical instrument 21, 22 by applying a radial constraint on the medical instrument 21, 22. Accordingly, the blocking wings 412 may move into and out of engagement with, for example, the shaft of a medical instrument extending through the common axial lumen 411, 421 of system 4, simply by screwing or unscrewing the female member 42 onto the male member 41. Also, in this non-restrictive exemplary embodiment of the disclosure, the plurality of intermediate positions in which the locking means 412, 427 can be set is tantamount to a homogeneous continuity of intermediate positions resulting from the cooperative continuity between the threads 416, 426 and the locking means 412, 427. This disposition offers, among other advantages, the possibility for the surgeon to easily replace a medical instrument of one type, initially held in the system according to the disclosure, by another instrument of another type.

Furthermore, in the example depicted in the figures, two fixation hooks 415 are disposed on the annular wall 414 (as shown in FIGS. 4B and 4C), for reversibly attaching the male member 42, and thereby reversibly attaching the system 4, to a trocar 3. In that manner, the system can be disposed after use, and easily replaced, if needed, by a new one.

In other embodiments of the disclosure, not depicted here, the attachment between a trocar and at least one member of the system, be it male and/or female, may be irreversible.

Further, according to one or more embodiment, the system may further comprise a gas tight gasket between the male member and the female member.

## Claims

1. System (4) for holding a medical instrument (21; 22) comprising:
a male member (41) comprising at least one male thread (416) and at least one first axial lumen (411);
a female member (42) comprising at least one female thread (426) and at least one second axial lumen (421);
wherein, when the at least one male thread (416) and the at least one female thread (426) are engaged to each other, the at least one first axial lumen (411) and the at least one second axial lumen (421) form a common axial lumen (411, 421) for at least partially receiving a medical instrument (21; 22); and
wherein said system (4) further comprises:
a locking means (412, 427) for locking the medical instrument (21; 22) in the common axial lumen (411, 421) at a predetermined position.

2. System (4) according to claim 1, wherein the locking means (412, 427) is configured to set the system (4) in any of a plurality of intermediate positions between an open position, at which the medical instrument (21; 22) freely moves in the common axial lumen (411, 421), and a closed position, at which the medical instrument (21; 22) is locked at the predetermined position.

3. System (4) according to claim 1 or claim 2, wherein the locking means (412, 427) is configured to apply at least one of a radial constraint and an axial constraint on the medical instrument (21; 22), and combinations thereof.

4. System (4) according to any of the preceding claims, wherein the locking means (412, 427) is integrally formed with the male member (41) and/or the female member (42).

5. System (4) according to any of the preceding claims, wherein the locking means (412, 427) comprises at least one of a blocking wing, a beveled cylindrical wall, a jaw, a wedge, a spring, and combinations thereof.

6. System (4) according to any of the preceding claims, wherein the male member (41) is reversibly attachable to a trocar (3) or integrally forms part of a trocar.

7. System (4) according to any of the preceding claims, further comprising a gas-tight gasket between the male member (41) and the female member (42).

8. Method for locking a medical instrument (21; 22) in a trocar (3), comprising:
providing the system (4) according to any of claims 1-7;
at least partially inserting a medical instrument (21; 22) in the common axial lumen (411, 421) of the system (4);
engaging the at least one male thread (416) and the at least one female thread (426) to each other to hold the medical instrument (21; 22) in a trocar (3); and
locking the at least partially received medical instrument (21; 22) in the common axial lumen (411, 421) at a predetermined position.
